(19) Europäisches Patentamt
European Patent Office
Office européen des brevets

(11) Numéro de publication: **0 278 845**
**A2**

# (12) DEMANDE DE BREVET EUROPEEN

(21) Numéro de dépôt: **88400203.1**

(22) Date de dépôt: **29.01.88**

(51) Int. Cl.⁴: **C 07 C 37/02**
C 07 C 39/15

(30) Priorité: **12.02.87 FR 8701967**

(43) Date de publication de la demande:
**17.08.88 Bulletin 88/33**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Nonn, Alain**
**1, allée de la Gravière**
**F-69110 Sainte-Foy-Les-Lyon (FR)**

**Desmurs, Jean-Roger**
**La Jonquière Route de Ternay**
**F-69360 Communay (FR)**

(74) Mandataire: **Le Pennec, Magali et al**
**RHONE-POULENC INTERSERVICES Service Brevets Chimie 25, Quai Paul Doumer**
**F-92408 Courbevoie Cédex (FR)**

## (54) Procédé de préparation d'hydroxybiphenyles.

(57) La présente inventin concerne un procédé de fabrication d'hydroxybiphényles par hydrolyse de bromobiphényles à une température inférieure à 250°C en présence de catalyseur à base de cuivre et d'une base alcaline ou alcalinoterreuse, caractérisé par le fait que la concentration en ions hydroxyle est inférieure à 2,5 équivalents d'hydroxyle par litre de solution.

EP 0 278 845 A2

**Description**

PROCEDE DE PREPARATION D'HYDROXYBIPHENYLES

La présente invention concerne un procedé de fabrication d'hydroxybiphényles. Elle concerne plus particulièrement un procédé de préparation de dihydroxy-4,4'biphényle.

En effet le dihydroxy-4,4'biphényle est un intermédiaire recherché actuellement dans l'industrie des matériaux synthétiques tels que les polyesters, les polyépoxides, les polyuréthanes, comme anti-oxydant dans les résines ou dans l'industrie des matières colorantes.

Il est connu de préparer les hydroxybiphényles par hydrolyse des halogénobiphényles à l'aide d'une catalyse au cuivre, telle que par exemple à l'aide de sulfate de cuivre (selon le brevet US 4 4475 500) ou à l'aide d'oxyde de cuivre (selon le brevet US 4 340 748) en milieu basique aqueux. Les conditions de température et la pression autogène créée par des températures aussi élevées ainsi que la faible quantité de matière première traitée à chaque opération ont incité l'homme de l'art à chercher des techniques plus douces nécessitant des températures plus modérées et donc permettant d'utiliser un appareillage moins onéreux.

Il est encore connu de nombreux procédés de préparation de composés hydroxyaryliques réalisés en phase vapeur, c'est à dire à une température supérieure à 300° C en présence de catalyseur à base de cuivre et d'un catalyseur à base de phosphates de terres rares. Il est ainsi décrit dans le brevet US 3 752 878 un procédé de préparation de composés hydroxyaryliques dont le groupe aryle contient un ou plusieurs noyaux benzèniques à partir de composés équivalents halogénés par hydrolyse en présence d'un catalyseur à base de phosphate de lanthane ou de cerium et de cuivre qui sont précipités conjointement sous forme de polyphosphate minéral. Cette réaction est réalisée sous forme biphasique. Le catalyseur se trouvant sous forme solide et le composé halogèné à hydrolyser sous forme gazeuse car la réaction est effectuée à 500° C. Le taux de conversion du coposé de départ est faible et les conditions de réaction sont trop sévères pour être utilisables à l'échelle industrielle.

Lorsque l'on désire réaliser l'hydrolyse à une température moins élevée, telle que par exemple selon le brevet japonais 69/17372 où l'hydrolyse est réalisée à environ 200° C, en présence de dérivés du cuivre à l'état d'oxydation deux, comme le sulfate de cuivre, le dibromure de cuivre, le diacétate de cuivre ; la durée de la réaction d'hydrolyse est particulièrement longue et atteint parfois 10 heures pour un rendement ne dépassant pas 25 %. Cette méthode n'est donc pas transférable au niveau industriel.

Ainsi depuis de nombreuses années l'industrie est à la recherche d'un procédé de préparation d'hydroxybiphényles économiquement rentable, non dangeureux à partir d'halogénobiphényles.

La présente invention a permis d'atteindre cet objectif et a pour objet un procédé industriel de préparation d'hydroxybiphényles par mise en contact en phase liquide aqueuse à une température inférieure à 250° C d'un bromobiphényle de formule générale (I) :

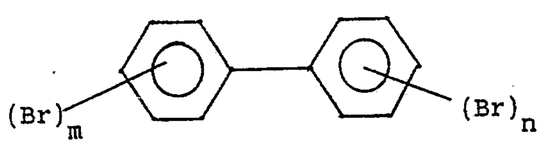

dans laquelle m et n sont des nombres entiers identiques ou différents, égal à 0,1, 2 ou 3 et dont la somme n+m est supérieure ou égale à 1 et inférieure ou égale à 4, avec une base de formule $M(OH)_p$ dans laquelle M est un métal choisi parmi les métaux alcalins ou alcalino-terreux, p est un nombre entier égal à 1 ou 2 suivant la valence du métal M et en présence d'un catalyseur à base de cuivre, caractérisé en ce que la base est utilisée à une concentration calculée en équivalents d'ions hydroxyle par litre de solution aqueuse comprise entre 0,1 et 2,5 équivalents molaires par litre et de préférence entre 0,1 et 1,5.

On peut schématiser la réaction sur le dibromobiphényle par exemple par l'équation réactionnelle suivante :

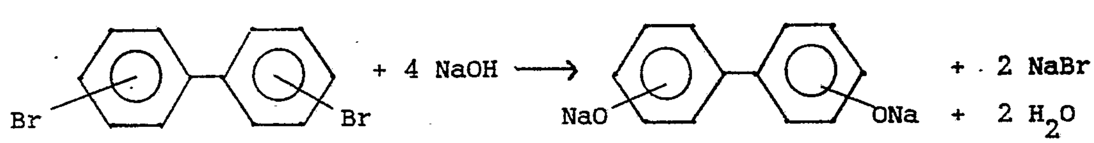


Il faut donc pour obtenir une réactivité correcte de la matière première avoir und solution contenant une quantité de base permettant de fournir deux radicaux hydroxyle pour chaque atome de brome à hydrolyser.

Par contre, la demanderesse s'est aperçue que la solution ne doit pas être trop concentrée en base sinon le rendement réactionnel diminue fortement. Ainsi une concentration de 2,5 équivalents molaires d'ions hydroxyle par litre est maximale. Dans la formule $M(OH)_p$ si p = 1 une concentration molaire de 2,5 moles par litre maximale comme par exemple, pour la soude et/ou la potasse. Pour une base où p = 2 une concentration

molaire de 1,25 mole par litre sera maximale.

On préfère utiliser une quantité de base telle que la concentration minimale en ions hydroxyle soit néammoins d'au moins 0,1 équivalent d'ions hydroxyle. Cette concentration minimale peut être maintenue par ajout continu de la base. On peut également introduire en continu le bromobiphényle. L'ajout continu de base est de préférence un ajout de base concentrée de l'ordre de 10 N ce qui évite de trop diluer le milieu réactionnel. Cet ajout de base doit maintenir le milieu réactionnel dans les limites précédemment définies.

L'avantage d'utiliser des solutions relativement diluées en base est de traviller avec un faible excès de base ce qui évite l'utilisation de grandes quantités d'acide lors de la neutralisation.

Les dérivés bromés sont préparés de manière connue par tout homme de l'art par action du brome sur un biphényle à température ambiante pendant quelques heures. La variation de température, de durée, le choix du solvant et le choix de certains catalyseurs de bromation permettent de fixer un ou plusieurs atomes de brome.

Parmi les dérivés de formule générale (I), on préfère les dérivés dans lesquels $n+m$ est égal à 1 ou 2 et encore plus préférentiellement ceux pour lesquels $n+m$ est égal à 2 et tout particulièrement le dibromo-4,4'biphényle.

Parmi les bases de formule $M(OH)_p$, on préfère utiliser les bases fortes alcalines et tout particulièrement la soude ou la potasse.

Parmi les catalyseurs à base de cuivre, on peut citer non limitativement le cuivre métallique, le cuivre à l'état d'oxydation un tel que l'oxyde cuivreux, les halogénures, acétate, cyanure, thiocyanate, triflate, sulfure cuivreux, ainsi que les dérivés du cuivre à l'état d'oxydation deux tels que l'oxyde, les halogénures, acétates, acétylacétonates, métaborate, isobutyrate, citrate, cyclohexylbutyrate, diméthyldithiocarbamate, hexanoate, gluconate, hydroxyde, oxalate, propionate, stéarate, sulfate, trifluoroacétylacétonate cuivriques.

On préfère cependant utiliser les oxydes ou halogénures de cuivre à l'état d'oxydation un ou deux.

Il peut être encore avantageux, et c'est un autre objet de la présente invention, d'ajouter un cocatalyseur choisi parmi

- les halogénures (fluorures, bromures),
- les phosphates,
- les nitrates,
- les alcoolates,
- les silicates minéraux ou organiques ainsi que leurs précurseurs,
- les dérivés soufrés organiques,
- l'oxyde de carbone ou un de ses précurseurs,
- le méthanol,
- les quinoléines,
- les ammoniums quaternaires,
- les amines tertiaires dont la triéthylamine, les pyridines,
- les acides sulfoniques,
- les phosphines, les phosphoniums,
- le palladium.

Parmi les co-catalyseurs permettant de réduire soit le température, soit la durée de la réaction, on peut choisir les halogénures, les phosphates, les nitrates, les silicates, l'ensemble de ces composés sont pour leur mise en oeuvre associés à des protons ou à des cations alcalins, alcalino-terreux, métalliques par exemple de cuivre, d'argent ou organiques. Les silicates peuvent aussi être préparés in-situ dans le milieu réactionnel par action par exemple de la base forte sur de la silice ou du verre. Les phosphates peuvent également être préparés in-situ à partir de phosphates organiques.

Parmi les co-catalyseurs entièrement organiques on peut également citer par exemple les alcoolates et les alcools contenant de préférence 1 à 12 atomes de carbone, les acides carboxyliques contenant 2 à 12 atomes de carbone, les acides sulfoniques, par exemple l'acide phényl ou pyridine sulfonique, les amines tertiaires et les ammoniums, les phosphines et les phosphoniums, les quinoléines, les dérivés soufrés ainsi que l'oxyde de carbone ou un de ses précurseurs organiques tels que les formiates.

Ainsi, on peut citer pour exemplifier les co-catalyseurs de la présente invention, le diphénylsulfure, le dithiophène, le fluorure de potassium et/ou de sodium, l'acide phosphorique, l'acide nitrique, le méthylate de sodium, le formiate de méthyle, les verres, par exemple le Pyrex, la silice, l'orthoformiate de méthyle, de méthanol, l'hydroxy-8 quinoléine sulfonique 5, le bromure de tétrabutylammonium, la triéthylamine, la pyridine, l'acide pyridine sulfonique, l'acide phénylsulfonique, le chlorure de tétraphénylphosphonium, la triphénylphosphine, la triphénylphosphinetrisulfonée, l'oxyde de triphénylphosphine, la tributylphosphine, la tricyclohexylphosphine, le chrome héxacarbonyle, la mousse de palladium, la N-méthylpyrrolidone.

On préfère utiliser les fluorures, les bromures, les phosphates, les nitrates, les formiates ou les silicates alcalins, les formiates organiques, les hydroxy 8 quinoléines.

Parmi les conditions réactionnelles, la température est une condition essentielle du point de vue économique et elle fait l'objet du présent procédé, elle est limitée à 250°C et de préférence comprise entre 180 et 230°C et encore plus préférentiellement entre 200 et 215°C. La pression utilisée est la pression autogène obtenue à la température de réaction par vaporisation des composés présents. Elle est notamment comprise entre 15 et 25 bars. Le durée de réaction varie avec la température utilisée. Elle varie néanmoins entre environ 2 heures et 5 heures. La réaction ayant lieu en milieu aqueux, on utilise de préférence un mono

ou poly- bromobiphényle à une concentration supérieure à 0,15 mole par litre d'eau et de préférence comprise entre 0,15 et 2 moles par litre d'eau.

La quantité pondérale de catalyseur à base de cuivre est de préférence comprise entre 0,2 % et 5 % par rapport à la quantité du bromobiphényle introduite.

La quantité de cocatalyseur est de préférence comprise entre 0,1 et 100 fois la quantité molaire de catalyseur. Une quantité largement excédentaire ne nuirait pas au procédé de l'invention mais cela n'apporte aucun avantage supplémentaire. La quantité utile sera simplement adaptée par l'homme de l'art à l'économie du procédé.

L'invention permet de préparer des hydroxybiphényles et parmi ceux-ci on peut citer ; l'hydroxy-4 biphényle, l'hydroxy-2 biphényle, l'hydroxy-3 biphényle, le dihydroxy-4,4'biphényle, le dihydroxy-2,2'biphényle, le dihydroxy-2,4'biphényle, le trihydroxy-2,4,4'biphényle, les tétrahydroxybiphényles.

L'invention sera plus complètement décrite à l'aide des exemples suivants qui ne doivent en aucun cas être considérés comme limitatifs de l'invention.

Dans les exemples suivants on entend par RR le rendement calculé sur le produit introduit dans le réacteur.

EXEMPLES COMPARATIF 1 A COMPARATIF 4 :

Mode opératoire :

On introduit dans un réacteur en Hastelloy C 276 de 75 ml les composés suivants :

- 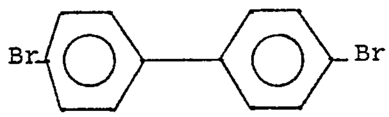 2 g (6,4 mmoles)
- $Cu_2O$ 10 mg (0,07 mmole)
- NaOH selon les concentrations et quantités explicitées dans le tableau ci-joint

Après une purge à l'argon, fermeture de l'autoclave, ce dernier est placé dans un four agité et porté à la température indiquée dans le tableau 1. Après une durée de réaction indiquée dans le tableau 1, le réacteur est ramené à température ambiante. Le mélange réactionnel est dilué par 20 ml d'eau (rinçage du réacteur), acidifié par 25 ml d'acide sulfurique 4N et extrait à la méthylisobutylcétone (1 x 20 ml + 5 x 10 ml). La phase organique est séchée sur sulfate de sodium. Les rendements sont déterminés par chromatographie liquide haute performance.

Tableau 1

| Exemples Comparatifs | NaOH concentration | T°C | Temps h | Mono OH | Di OH |
|---|---|---|---|---|---|
| Comparatif 1 | 5,0 N (20 ml) | 250 | 2 | 0,3 | 39 |
| Comparatif 2 | 2,5 N (20 ml) | 250 | 2 | 1,5 | 78,6 |
| Comparatif 3 | 5,0 N (20 ml) | 230 | 3 | 0,05 | 15 |
| Comparatif 4 | 2,5 N (20 ml) | 230 | 3 | 0,3 | 29,8 |

EXEMPLES 1 et 2 - Selon l'invention :

On introduit dans le même réacteur que précédemment :

- 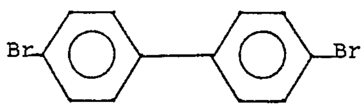 2 g (6,4 mmoles)

- $Cu_2O$ 10 mg (0,07 mmole)

- NaOH 1 N 30ml (30 mmoles)

| Exemples | T°C | Temps h | Mono OH | Di OH |
|---|---|---|---|---|
| 1 | 250 | 2 | 5,8 | 84,5 |
| 2 | 230 | 3 | 4,1 | 82,4 |

EXEMPLES SELON L'INVENTION N° 3 à 4

On introduit dans un réacteur à agitation centrale par turbine de Rushton (800 tours/mn) les composés suivants :

- dibromo-4,4'biphényle = 20 g (64 moles)
- $Cu_2O$ = 100 mg (0,7 mmoles)

| Exemples Comparatifs | NaOH concentration | T°C | Temps h | Mono OH | Di OH |
|---|---|---|---|---|---|
| Comparatif 5 | 2,5 N (200 ml) | 230 | 2 | 1,5 | 70 |
| Comparatif 6 | 2,5 N (200 ml) | 215 | 3 | 0 | 11,7 |
| 3 | 1,0 N (300 ml) | 230 | 2 | 5,7 | 82,4 |
| 4 | 1,0 N (300 ml) | 215 | 3 | 4,7 | 81,6 |

EXEMPLES SELON L'INVENTION 5 à 8

Dans le même appareillage que prédédemment on introduit :

- 20 g de dibromo-4,4' biphényle (64 mmoles)
-100 mg de $Cu_2O$ (0,7 mmole)
-Température de réaction = 215°C.

TABLEAU 4

| Exemple Comparatif | Durée de réaction avant addition | Volume NaOH 10 N ajouté | Durée de réaction | NaOH total (moles) | Durée totale | RR mono OH | RR di OH |
|---|---|---|---|---|---|---|---|
| Comparatif 7 | - | - | - | 0,2 | 3 h | 0,1 | 53,1 |
| Comparatif 8 | - | - | - | 0,2 | 2 h | 0,3 | 47,8 |
| 5 | 1 h | 30 ml | 1 h | 0,5 | 2 h | 0,6 | 56,1 |
| 6 | 0,75 h | 5 ml | 0,25 h | - | - | - | - |
| | - | 5 ml | 0,75 h | 0,3 | 2 h | 0,7 | 57,9 |
| 7 | 0,75 h | 5 ml | 0,50 h | - | - | - | - |
| | - | 5 ml | 0,25 h | - | - | - | - |
| | - | 5 ml | 0,50 h | 0,35 | 2 h | 0,8 | 61,2 |
| 8 | 1 h | 5 ml | 0,70 h | - | - | - | - |
| | - | 5 ml | 0,70 h | - | - | - | - |
| | - | 5 ml | 0,70 h | 0,35 | 3 h | 1,6 | 81,8 |

EXEMPLE 9

Charge :
20 g de dibromo-4,4′ biphényle (0,064 mole)
300 ml de NaOH 1 N (0,300 mole)
100 mg $Cu_2O$
Conditions :
200° C agitation
3 H 800 tours/mn.

| Additif * | RR (%) 4-OH | RR (%) 4,4'diOH | TT diBr |
|---|---|---|---|
| Sans | 0,9 | 35,0 | 44,6 |
| Hydroxy-8 quinoléïne | - | 57,7 | - |

* 2 moles/mole de catalyseur.

**Revendications**

1°) Procédé de préparation d'hydroxybiphényles, par mise en contact en phase liquide aqueuse à une température inférieure ou égale à 250° C, d'un bromobiphényle de formule générale (I) :

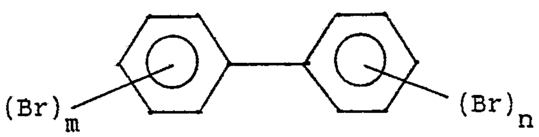

dans laquelle m et n sont des nombres entiers identiques ou différents égal à 0,1, 2 à 3 et dont la somme n+m est supérieure ou égale à 1 et inférieure ou égale à 4, avec une base de formule $M(OH)_p$ dans laquelle M est un métal choisi parmi les métaux alcalins ou alcalino-terreux, p est un nombre entier égal à 1 ou 2 suivant la valence de métal M en présence d'un catalyseur à base de cuivre caractérisé en ce que la base est utilisée selon une concentration dans le milieu réactionnel en ions hydroxyle comprise entre 0,1 et 2,5 équivalents molaires/1, et de préférence comprise entre 0,1 et 1,5 équivalents molaires/1.

2°) Procédé selon la revendication 1, caractérisé en ce que dans la formule (I) n+m est égal à 1 ou 2.

3°) Procédé selon la revendication 1 et 2, caractérisé en ce le composé de formule (I) est le dibromo-4,4′biphényle.

4°) Procédé selon la revendication 1, caractérisé en ce que le catalyseur à base de cuivre est choisi parmi les oxydes ou les halogénures du cuivre à l'état d'oxydation un ou deux.

5°) Procédé selon la revendication 1, caractérisé en ce que la concentration dans le milieu réactionnel en ions hydroxyle est maintenue entre 0,1 et 2,5 équivalents/1 par ajout continu de base et de préférence entre 0,1 et 1,5 équivalents par litre.

6°) Procédé selon l'une quelconque des revendications précédentes, caractérisé en ce que l'on introduit en continu le bromobiphényle et la base.

7°) Procédé selon la revendication 1, caractérisé en ce que parmi les conditions réactionnelles, on opère à une température comprise entre 180 et 230° C et de préférence comprise entre 200 et 215° C.

8°) Procédé selon la revendication 5, caractérisé en ce que l'ajout continu de base est un ajout de base environ 10 N.

9°) Procédé selon la revendication 1 caractérisé en ce qu'on ajoute un cocatalyseur choisi parmi les fluorures, les phosphates minéraux ainsi que leurs précurseurs organiques, les nitrates, les alcoolates, les silicates minéraux ou organiques ainsi que leurs précurseurs, les dérivés organiques soufrés, les

alcools, les acides carboxyliques, l'oxyde de carbone ou un de ses précurseurs, les acides sulfoniques, les quinoléïnes, les ammoniums, les amines tertiaires ou les phosphines.

10°) Procédé selon la revendication 9 caractérisé en ce que le cocatalyseur est ajouté selon un rapport molaire calculé par rapport au catalyseur compris entre 0,1 et 100.